# EUROPEAN PATENT APPLICATION

(11) **EP 3 470 511 A1**
(43) Date of publication of application: **17.04.2019**
(21) Application number: 18200059.6
(22) Date of filing: 12.10.2018
(51) Int. Cl.: C12M 3/00, C12M 1/42, C12M 1/12, C12M 1/00

(54) **BIOREACTOR FOR THE RECELLULARIZATION AND MECHANICAL STIMULATION OF A DIAPHRAGM**

(30) Priority: 13.10.2017 IT 201700115761
(71) Applicant: Istituto di Ricerca Pediatrica Citta della Speranza, 35127 Padova (IT)
(72) Inventor: PICCOLI, Martina, 35127 Padova (IT); PAVAN, Piero, 35127 Padova (IT)
(74) Representative: Manfrin, Marta

(57) **Abstract**

A bioreactor (1) for the recellularization of a diaphragm, characterized by a radial distribution of the muscle fibers, comprising an incubator (2) which has a first region (3) suitable for containing a culture medium in which the diaphragm is to be immersed. The incubator also comprises a membrane (5) which is deformable along several directions when mechanically stressed and which has a first surface (7) which is directed towards said first region (3) and on which said diaphragm is intended to be supported. The bioreactor (1) also comprises means (14) for mechanically stressing the membrane (5). These means interact with a second surface (8) of the membrane (5) so as to provide during use the diaphragm with mechanical stimuli oriented along the direction of extension of said fiber muscles.

## Description

### Technical field of the invention

The present invention falls within the sector of regenerative medicine and, more specifically, tissue engineering for the generation of muscle tissues *in vitro* and relates to a bioreactor for the recellularization and controlled mechanical stimulation of a decellularized muscle, in particular of a diaphragm.

### Background

Tissue engineering is a multidiscipline research sector which employs the principles of biochemistry, medicine, engineering and physics in order to reproduce *in vitro* portions of biological tissues intended to replace human tissues which have been damaged by traumatic events or are affected by pathologies.

This sector has arisen in response to the growing demand for tissues and organs which cannot be satisfied by donations and the insufficient quantity of tissue available in the case of autotransplants, with the aim of overcoming all the problems of rejection which frequently arise in the case of tissue transplants (both allotransplants and xenotransplants) as well as the problems associated with serious viral infections which may be present in the materials of human or animal origin used for grafting in transplants.

More specifically, tissue engineering aims to achieve the culture, within laboratories and in many cases also on an industrial level, of cell lines and tissues which have the characteristics of the recipient. The cells and the tissues obtained *in vitro* are then grafted into the patient, thus restoring the damaged functionalities without having to resort to the transplantation of biological materials removed from foreign donors. Compared to conventional transplantation methods, therefore, the engineered tissues, in the event of a successful outcome, become integrated with those of the patient, thus helping cure in a specific and permanent manner the pathological condition without the need for costly and debilitating pharmacological treatment.

An example of an operation where the transplant of a tissue is required is that of the diaphragm muscle. This muscle may in fact be subject to congenital defects and malformations which adversely affect its functionality. Diaphragmatic hernia, for example, is a pathology which constitutes about 8% of all the congenital malformations in newborn babies.

Tissue engineering is based on the interaction between biological components (cells, culture medium) and biomaterials (substrate or scaffold), all of which are placed inside a culture chamber called a bioreactor which imparts to the cells seeded into the substrate the appropriate stimuli for achieving growth of a tissue functionalized *in vitro.* The bioreactor may be divided into two subsystems: the growth environment and the control system. The former is composed of a growth chamber inside which the substrate seeded with cells is housed. The control system is composed of the actuators, the sensors and a software which sends and receives signals from them. The system is therefore able to monitor and regulate one or more environmental variables (pH, temperature, oxygenation, metabolic activity of the cells) which characterize the biological processes in progress inside the growth chamber and, in the case of bioreactors which apply stimuli to the constructed assembly, also the physical variables such as the mechanical stress, electrical stimulation and magnetic stimulation. It is in fact known from the state of the art that the application of a mechanical stimulus to the substrate on which the cells are cultivated increases the capacity of the muscle precursors, both of human origin and of animal origin, to become aligned along the axis to which the stimulus is applied and favor the formation of new fibers and cell differentiation, both *in vitro* and *in vivo.* This observation is applicable also to the stimulation of differentiation and contraction in innovative culture systems, such as three-dimensional systems and engineered muscle tissues. For these reasons, in many bioreactors, the substrate is stretched and released with predefined frequency cycles such that the cell tissue placed on it is subject to similar periodic deformation cycles which favor the formation of new fibers and cellular differentiation.

The cell stimulation devices which are currently available are however designed to provide a mechanical stimulus in a single specific direction. This approach, however, is not optimal for all the types of tissues to be regenerated. More specifically, muscles which are characterized by a particular arrangement of the fibers, i.e. where the fibers are not all parallel to each other, require different types of stimuli. The diaphragm, for example, is characterized by muscle fibers arranged radially within space and requires stimulation which is compatible with this spatial arrangement, oriented as far as possible along the direction of formation of said muscle fibers.

At present there does not exist a cell stimulation device designed to provide a mechanical stimulus suitable for the regeneration of the diaphragm muscle. Consequently, if transplant of a diaphragm is required, there exists no cell stimulation device which is able to provide a mechanical stimulus compatible with the spatial arrangement of the fibers of this type of muscle.

### Summary of the invention

The object of the present invention is therefore to provide a device, in particular a bioreactor, which is able to overcome all the drawbacks mentioned above with reference to the prior art. This is obtained by means of a bioreactor and a method according to the independent claims. Secondary characteristics of the bioreactor according to the present invention are defined in the dependent claims.

In particular, in accordance with the present disclosure, a bioreactor is provided where the culture medium is arranged on the surface of a first deformable membrane. The first membrane is stressed to perform a movement such that the muscle fibers which form on the surface extend in the radial direction. The stressing is applied onto an opposite surface of the membrane, preferably in a direction perpendicular to the membrane.

Even more particularly, stressing means which include a sealed body filled with a fluid and an actuator are provided, said sealed body comprising a first chamber, a second chamber and a connection duct for connecting the first chamber to the second chamber. The first membrane is a wall of said first chamber and separates the first region from the first chamber, wherein said second chamber includes at least a respective second membrane or a respective flexible and/or deformable wall. The actuator is designed to interact on an outer side of the second membrane of the second chamber so as to produce a displacement of the fluid inside the sealed body from the second membrane to the second surface of the first membrane of the first chamber so as to produce said mechanical stimuli, and more particularly, produce a displacement of the fluid.

Preferably, the first chamber and the second chamber have a width greater than the connection duct. As a result of the wider first chamber a wider membrane for pushing towards the diaphragm may be obtained. As a result of the wider second chamber a greater exposure to the actuator may be obtained. The two membranes are therefore wider than the cross-section of the connection duct. In this way the connection duct acts only as a device for transferring the pressure wave for displacement of the fluid.

Even more preferably the connection duct is a flexible pipe. In this way an alignment between the first chamber and the second chamber of the bioreactor is not necessary. Also, they may be arranged anywhere, even at a great distance.

For example, the first chamber is situated inside the incubator, while the second chamber is situated outside the incubator. The actuator is also situated outside the incubator. This embodiment offers the advantage that the temperature conditions inside the incubator may be safeguarded.

Stressing is preferably obtained by means of an actuator which performs a to-and-fro movement with respect to the membrane. In this way two bioreactors may also be provided, both being connected to the same actuator. The actuator stresses the first membrane of a first bioreactor when it moves in one direction and stresses the first membrane of the other bioreactor or second bioreactor when it moves in a second direction.

The advantages, as well as the characteristic features and the modes of use of the present invention will emerge clearly from the following detailed description of preferred embodiments thereof, provided by way of a non-limiting example, with reference to the figures of the attached drawings in which:
- Figure 1 shows a schematic diagram of the bioreactor according to the present invention;
- Figure 2 shows a diagram of the bioreactor according to the present invention where the membrane which supports the diaphragm is not deformed;
- Figure 3 shows the bioreactor according to the present invention where the membrane which supports the diaphragm is deformed;
- Figure 4 shows a second embodiment of the bioreactor for the simultaneous stimulation of two diaphragms.

The present invention, by overcoming the aforementioned problems, gives rise to numerous obvious advantages.

Generally, the use of the bioreactor according to the present invention in regenerative medicine is widely adopted as a tissue engineering technique for the generation of muscle tissue *in vitro* and for assessing the therapeutic effects of drugs and molecules aimed at treating and slowing down myopathies in particular affecting the diaphragmatic muscle. With this invention it is in fact possible to construct a replacement diaphragm relatively quickly in order to allow repair of the native tissue subject to damage and/or defects. The diaphragm obtained will allow for example the restoration of the functionality of this muscle in newborn babies suffering from diaphragmatic hernia, or repair of the tissue following damage due to traumas or accidents also in the case of adults.

Unlike the cell stimulation devices which are currently available, the bioreactor according to the present invention is able to produce a mechanical stimulus compatible with the spatial arrangement of the fibers of the muscle to be regenerated, in particular the diaphragm. The stimulation imparted to the substrate by the commonly available reactors is in fact not suitable for the regeneration of muscles which are characterized by a particular spatial arrangement of the muscle fibers. Unidirectional stimulation of the diaphragm, for example, is not optimal for the regeneration of the its muscle fibers, which are essentially arranged radially.

The mechanical stimuli induced by the bioreactor according to the present invention are able to promote the growth of muscle fibers with an orientation similar to that of the muscle fibers of a native diaphragm. The mechanical stressing is moreover controlled in terms of its intensity, frequency and duration so as to maximize the effects of the stimuli on the growth of the muscle fibers.

Finally, the bioreactor according to the present invention is useful also for studies aimed at identifying the origin and progression of different types of myopathies (for example Duchenne muscular dystrophy) where the mechanical stress, to which the diaphragm is physiologically subjected, causes an accentuation and acceleration of the effects of the illness. It is thus possible to recreate in a laboratory a diaphragm ex-novo from a decellularized matrix and muscle cells of the patient. As a result of this invention it is possible to study the degeneration mechanisms, the effects and the drug doses needed to repair or slow down the advance of the illness. This type of application may also be useful as "personalized medicine" and be used therefore routinely for determination of the doses of molecules and therapeutic agents.

Other advantages, characteristic features and modes of use of the present invention will become clear from the following detailed description of a number of embodiments, provided by way of a non-limiting example.

### Detailed description of preferred embodiments

Below various embodiments and variants of the invention will be described with reference to the figures mentioned above.

The different embodiments and variants described below may be used in combination, where compatible.

The present invention relates in particular to a bioreactor for the recellularization and mechanical stimulation of a decellularized diaphragm. The bioreactor in question, in particular, is designed so that the mechanical stimulus to the diaphragm is provided by the controlled deformation of a membrane with a specific morphology to which the diaphragm is attached.

With reference to the figures, the bioreactor according to the present invention is denoted overall by 1.

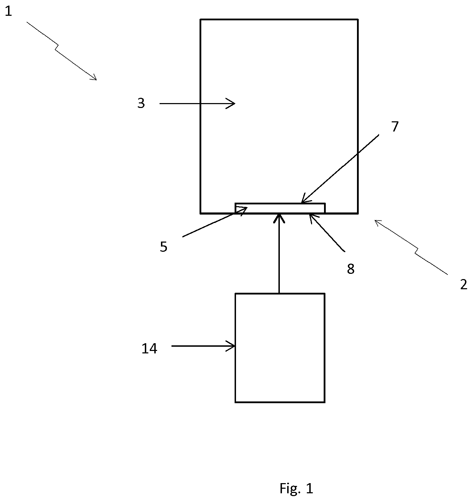

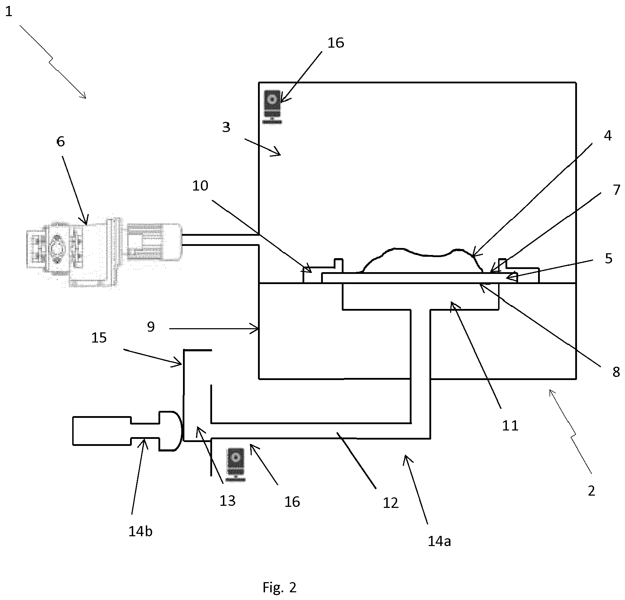

More specifically, as schematically shown in Figure 1 and in greater detail in Figure 2, the bioreactor 1 comprises an incubator 2 kept under a controlled atmosphere and having, internally, a region 3 intended to contain the diaphragm 4, the first membrane 5 supporting the latter and stressed by special mechanical stressing means, and the culture medium suitable for cell growth.

A system comprising a pump 6 controlled by special software ensures replacement of the culture medium inside the region 3. The maintenance and/or replacement of the culture medium may therefore be regulated depending on planning of the mechanical stimulus to be applied to the diaphragm and vice versa. The system envisages moreover circuits and valves for emptying and filling the space available for the diaphragm. Said maintenance and/or replacement system may operate both during decellularization of the diaphragm and during cell growth, ensuring the provision of the solutes needed for optimum evolution of the two stages of the process.

The conditions inside the region 3 containing the culture medium are controlled by means of the normal sensor system provided for this kind of apparatus, which continuously monitors the levels of temperature, CO₂ and relative humidity.

The diaphragm 4 is attached to a first surface 7 of the first membrane 5 situated inside the region 3 of the incubator 2. The positioning of said first membrane 5 must be such as to ensure that the diaphragm 4 is completely immersed within the culture medium in order to ensure cell growth. More specifically, the first membrane 5 is attached to a bottom support 9 of the incubator 2, preferably made of PTFE or equivalent material, by a retaining ring 10.

The first membrane 5 to which the diaphragm 4 is fixed is made of elastomeric material. Depending on the method used to fix the diaphragm 4, the latter may consist of one or more layers of PDMS or other material with an elastic behaviour which is similar and has the same biocompatibility. Said elastic membrane 5 must moreover be at the same time more rigid than the diaphragm 4 supported by it so as to allow precise control of the actual mechanical stimulus imparted to the latter. Given the rigidity ratios, in fact, the diaphragm 4 is necessarily deformed in accordance with the deformation of the first membrane 5. More specifically, the membrane rigidity in mono-axial conditions of the muscle tissue which forms the diaphragm 4 is equal to about 0.02-0.045 N/mm in the mouse specimen, while that of the membrane 5 is at least 1 N/mm. The value of the membrane rigidity of the element 5 is determined based on the value of the expected membrane rigidity of the diaphragm, depending on the animal model considered. The dimensions and the shape of the latter must be such as to adapt to the morphology of the diaphragm 4. In particular, the shape is preferably circular, while the diameter may be variable within a wide range, depending on the dimensions of the diaphragms which are to be subjected to the recellularization and mechanical stimulation treatment. Finally, since the anatomy of the diaphragm member is such as to define a central tendon which is of no interest for the mechanical stimulation, the membrane 5 may have a variable thickness which is thinner in the region of the muscle tissue and thicker in the region of the central tendon. In this way, it is possible to maximize the induction of the mechanical stimulus to the muscle part of the diaphragm member.

As mentioned above, the stimulation of the diaphragm muscle 4 is performed by means of stressing of the first membrane 5 by special mechanical stressing means.

In particular, in the bottom support 9 of the incubator 2 there is a first sealed chamber 11, separated from the region 3 of the incubator 2 by the first membrane 5 and directed towards the second surface 8 of the latter. The first membrane 5 is therefore a wall of the first chamber 11. The membrane 5 separates the first chamber from the first region and/or prevents direct contact between the first chamber and the first region.

This first chamber 11 is filled with fluid and is able to transfer a stimulus from a stimulus producing means to the membrane 5. In particular, the deformation of the membrane 5 is obtained by means of induction of a hydrostatic pressure in the fluid contained in the first chamber 11.

A special pipe 12, together with a series of valves/suitable for introducing and emptying the filling fluid of the first chamber 11 connects the latter to a second chamber 13. Said second chamber 13 has in turn a second membrane 15 designed to interact with the mechanical stressing means in order to produce a pressure wave intended to deform the membrane 5. It can therefore be seen that the first chamber 11, the second chamber 13 and the pipe 12 form a sealed body containing the fluid. The fluid is present continuously between the second chamber, the pipe 12 and the first chamber 11.

More specifically, an increase of the pressure inside said second chamber 13 is obtained by means of the action of one or more linear actuators 14b on the second membrane 15 of said second chamber 13. The pressure wave generated in this second chamber 13 is then transferred from the pipe 12 to the first chamber for deformation of the membrane 5 which, in turn, stimulates mechanically the diaphragm 4.

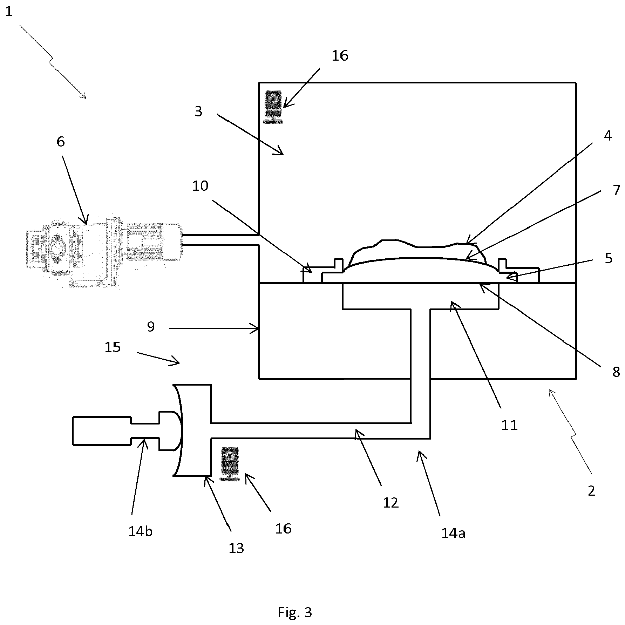

As can be seen in Figure 3, therefore, the action of the linear actuator 14b causes an increase in pressure inside the second chamber 13 which propagates towards the first chamber 11 via the pipe 12. The increase in pressure inside said first chamber 11 deforms the membrane 5 which, in turn, stimulates the diaphragm 4 attached to it.

Owing to the separation by the membrane 5 of the region 3 inside which the diaphragm is placed, from the linear actuator 14b, the diaphragm 4 may be placed inside any incubator, ensuring ideal conditions of sterility, temperature and level of CO₂ for maintaining in vivo conditions of the tissue. In fact, the actuator 14b and the second chamber 13 may be placed outside the incubator 2, without adversely affecting the temperature conditions inside the latter. The pipe 12, or connection duct, may be inserted sealingly inside a through-hole formed in a wall of the incubator. The pipe 12 is preferably flexible. In this way alignment of the first chamber with the second chamber is not necessary. In general, the arrangement of the components of sealed body is greatly facilitated. Moreover, it should be pointed out that the actuator is a device separate from the sealed body 14 and therefore, owing to the flexible pipe, the position of the second chamber may be adapted to the position of the actuator 14b.

The linear actuator 14b which induces the deformation inside the second chamber 13 may be alternatively replaced by a servo motor with a circular movement connected to a connecting rod / crank system. It may also be replaced by a pump which can be controlled in a similar manner by a dedicated electronic board and by a specific software. Said software is installed in a normal personal computer, which may also be a portable PC, and allows programming of the pressure inside the second chamber 13 and therefore inside the first chamber 11 and deformation of the membrane 5 with cycles of a predefined duration which may be varied in terms of intensity and frequency in a fully programmable manner. It is also possible to generate pressure cycles with an intensity and frequency variable in a random manner. The subsequent mechanical stimulus on the diaphragm 4 is controlled in terms of intensity, frequency and form of the cycle as well as overall duration of the stimulus.

Owing to the fact that the fluid is situated inside a sealed body, such control of the intensity, frequency and form of the cycle and the overall duration of the stimulus may be performed easily without removing the fluid from the sealed body. In other words, the configuration of a sealed body allows the fluid to be used as a system for transferring a movement of the actuator 14b. And this transfer of fluid may occur in a cyclical manner, with a frequency and/or intensity which is/are determined beforehand The two chambers and the respective membranes have instead the function of receiving and transferring the impulse.

Moreover, it should be pointed out that the first chamber 11 and the second chamber 1 have a breadth which is greater than that of the pipe 12. In this way the second membrane 15 and the first membrane 5 have a surface which is wider than the cross-section of the pipe 12 and preferably sufficiently wide to receive the stimulus and transmit the stimulus to the diaphragm respectively. The connection pipe has solely the function of transferring the pressure wave for displacement of the fluid.

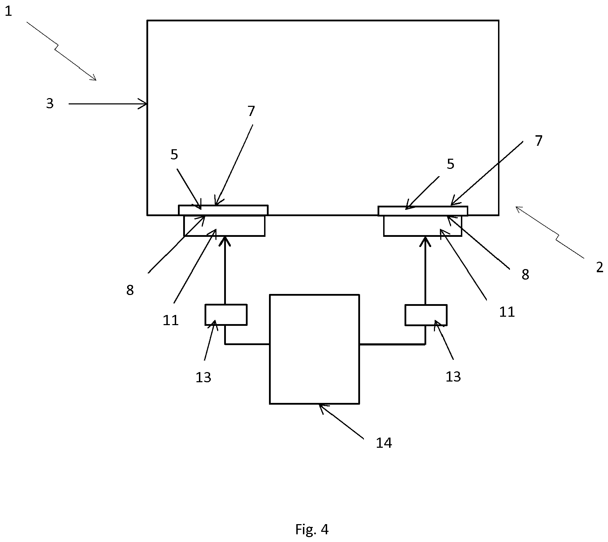

As schematically shown in Figure 4, the system described with regard to its main operating principles may be duplicated by using two second chambers 13 connected as described above to two first chambers 11 in a mutually independent manner. More specifically, the second chambers 13 are arranged in a diametrically opposite position to the linear actuator 14 so as to generate simultaneously two pressure cycles in separate circuits. This system is designed so as to be able to assess experimentally two diaphragms 4 or two diaphragm portions simultaneously and in the same ambient conditions, while varying, for example, the flowrate of the culture medium (increasing factors, etc.) or the deformation history in terms of the degree of deformation, for the same frequency. With this system it is also possible to vary the overall duration of the stimulus of the two diaphragms 4.

The parallel configuration of the system described above is obtained by arranging several linear actuators 14 and second chambers 13 in parallel. In this way, each linear actuator connected to one or two second chambers 13 controls up to two diaphragms which can be assessed as described above. The advantage of this configuration is that of being able to differentiate the deformation history of pairs of diaphragms, not only in terms of degree of deformation or duration of the stimulation, but also in terms of the frequency of induced stressing or the flowrate of the culture medium.

For the simultaneous stimulation of several diaphragms it is possible to provide combinations of first chambers 11 of a wide variety so as to be able to stimulate in the same conditions both diaphragms with the same "diameters" and diaphragms with substantially different "diameters". For example, two membranes with a nominal diameter of 22 mm and 24 mm, or eight membranes with a nominal diameter variable between 20 mm and 38 mm, with an interval of 2 mm, may be combined.

Alternatively, a same actuator may be used to stimulate first chambers 11 of a first bioreactor and second bioreactor, respectively, in an alternating manner. The actuator performs a to-and-fro movement and stimulates alternately the two first chambers. The presence of a flexible pipe facilitates this advantageous use of the to-and-from movement since the flexible pipe may be suitably bent as required in order to connect the actuator to the respective chamber inside the incubator.

The aforementioned software for controlling the linear actuator 14 was suitably programmed by means of numerical analyses carried out using the Finite Element Method (FEM) or similar methods on the degree of deformation imparted to the membrane 5 following the introduction of a suitable pressure level. As a result of these analyses it is possible to obtain a movement of the linear actuator 14 such as to provide the desired degree of variation over time of the pressure levels inside the first chamber 11 and the second chamber 13. The mechanical behaviour of the membrane 5 is also assessed by means of mechanical experimental tests designed to determine its tensile/deformation behaviour. By means of suitable constitutive laws introduced into the FEM program it is possible to obtain the correct description of the mechanical response of the membrane depending on the correct pressure conditions envisaged. The mechanical response of the diaphragm tissue is assessed in a similar manner. By means of specific constitutive laws it is possible to perform the *in silico* estimation of the deformation levels induced by the membrane on the diaphragm. The data of the FEM analysis is moreover necessary when the membrane is to be given a form in accordance with the morphological characteristics of the diaphragm.

The software, in addition to controlling the linear actuator 14 for the induction of the hydrostatic pressure state in the second chamber 13 and, therefore, in the first chamber 11, has a second main function: control of the pump 6 which ensures replacement of the culture medium inside the region 3.

Control of the maintenance and/or replacement of the culture medium is programmable in a manner synchronized with the mechanical stimulus, so that the replacement of the medium occurs in static mode, therefore with membrane and diaphragm not deformed. It is thereby possible to obtain an appropriate laminar regime of the flow of the culture medium, ensuring maximum homogeneity of diffusion of the solutes in the culture medium.

The bioreactor 1 is completed by sixteen cameras positioned so that the system may be monitored continuously and remotely. More specifically, these sixteen cameras are positioned so as to monitor what happens in the region of the diaphragm 4 and the first chamber 11 and in the region of the second chamber 13 and the linear actuator 14. In this way it is possible to control also remotely the beginning, the possible variation and the end of the stimulus and growth protocols of the diaphragmatic tissue, both as regards the mechanical aspect, i.e. the intensity and frequency of the deformation induced, and as regards the biological aspect, i.e. the maintenance and replacement of the culture medium. The sixteen cameras can be connected via cable or, more conveniently, wirelessly to a PC or smartphone.

In addition, in order to control, also remotely, any critical situations of the system during its operation and to be able to perform any corrective operations, the linear actuator 14 or the equivalent means for producing the deformation of the membrane 5 are monitored by sensors which send signals of any abnormalities to a control PC or a smartphone. More specifically, the linear actuator 14 or the other deformation producing means include temperature probes which continuously monitor the temperature of said deformation means.

Below are two examples of the application of the bioreactor according to the present invention.

### Example 1: Recellularization of decellularized mouse diaphragm with human precursors from muscle biopsy.

After obtaining the decellularization of a whole mouse diaphragm, the matrix is freed from the residual ribs and mounted and fixed on the PDMS membrane by means of small needles. With an injection method (Hamilton syringe with 33G needle) human muscle cells are injected (about 500,000 cells/mm2) into the matrix. The PDMS membrane with the recellularized diaphragm is then mounted on an incubator well, fixed with the corresponding glass and left to incubate for 2 hours and 30 minutes in an incubator with 5% CO₂ and 95% humidity. After this period, a culture medium (2-3 mL) suitable for cell proliferation is added to the well (DMEM low glucose - 1 g/l D-glucose - with 20% FBS, 10-6 M dexamethasone, 10 ng/mL bFGF, 10 µg/mL insulin, 1% penicillin/streptomycin). After 24 hours (or longer depending on the desired effects) the contraction is started with a cyclic protocol that provides for 30 BPMs (breaths per minute) and a deformation of 10% with respect to the rest phase.

### Example 2: Evaluation of muscle stem cell activation following chemical damage.

After isolating a diaphragm from a healthy mouse about 3 months old, the sample is mounted on the PDMS membrane, inserted into the incubator and fixed with a small glass so as to allow it to be anchored to the system. The diaphragm is then treated with a solution containing a toxin (cardiotoxin) which is widely used in the literature as a molecule capable of specifically destroying muscle fibers and activating accordingly the stem cells residing in the skeletal muscle. The toxin (0.2 µM) is left to act for 6 hours, at the end of this period the diaphragm is washed with PBS 1X and culture medium suitable for supporting the cell proliferation (DMEM low glucose - 1 g/l D-glucose - with 20% FBS, 10-6 M dexamethasone, 10 ng/mL bFGF, 10 µg/mL insulin, 1% penicillin/streptomycin) is added. Mechanical stimulation is then initiated, regulating alternately the number of BPMs and the percentage of membrane deformation. After 24 and 48 hours the diaphragm is removed from the bioreactor, fixed in 4% paraformaldehyde, dissected in a cryostat and analyzed using immunofluorescence and immunohistichemistry techniques for analysis of stem cell activation. It is therefore possible, using different animal models (e.g. healthy vs. myopathic) or with different experimental conditions (e.g. low BPM vs. high BPM) to understand how and when muscle stem cells are activated and to study therefore in detail and preicsely the mechanisms of regeneration of skeletal muscle.

The present invention has been described hitherto with reference to preferred examples of embodiment. It is understood that there may be other embodiments which relate to the same inventive idea, as defined by the scope of protection of the claims provided hereinbelow

## Claims

1. Bioreactor (1) for the recellularization of a diaphragm (4) **characterized by** a radial distribution of the muscle fibers, comprising:
- an incubator (2) comprising a first region (3) suitable for containing a culture medium in which said diaphragm (4) is to be immersed and a membrane (5) having a first surface (7) which is directed towards said first region (3) and on which the diaphragm is intended to be supported, said first membrane (5) being deformable along several directions when mechanically stressed;
- stressing means (14) for mechanically stressing said membrane (5) by means of the interaction with a second surface (8) of the membrane (5), opposite to the first surface (7), in order to provide during use the diaphragm (4) with mechanical stimuli oriented along the directions of extension of said muscle fibers,
wherein said stressing means (14) include a sealed body (14a) filled with a fluid and an actuator (14b), said sealed body (14a) comprising a first chamber (11), a second chamber (13) and a connection duct (12) for connecting the first chamber (11) to the second chamber (13),
wherein said first membrane (5) is a wall of said first chamber (11) and separates the first region (3) from the first chamber (11), wherein said second chamber (13) includes at least a respective second membrane (15) or a respective flexible and/or deformable wall;
wherein said actuator (14b) is designed to interact on an outer side of the second membrane (15) of the second chamber (13) so as to produce a displacement of the fluid inside the sealed body (14a) from the second membrane (15) to the second surface (8) of the first membrane of the first chamber (11) in order to produce said mechanical stimuli.

2. The bioreactor (1) according to claim 1, wherein said first membrane (5) and said second membrane (15) have a width greater than a cross-section of the connection duct (12).

3. The bioreactor (1) according to claim 1 or claim 2, wherein the connection duct (12) is a flexible pipe.

4. The bioreactor (1) according to any one of the preceding claims, wherein the first chamber (11) is situated inside the incubator (2), the second chamber (13) is situated outside the incubator (2), and said connection duct (12) is inserted inside a through-hole in a wall of the incubator (2).

5. The bioreactor (1) according to any one of the preceding claims, wherein the connection duct of the mechanical stressing means (14) is directed centrally or is centered with respect to said second surface (8) of the first membrane.

6. The bioreactor (1) according to any one of the preceding claims, wherein the membrane (5) has a circular shape.

7. The bioreactor (1) according to any one of the preceding claims, wherein the membrane (5) is made of elastomeric material, and preferably PDMS, and/or wherein the material that composes the membrane (5) has a membrane rigidity at least 20 times that of the stimulated diaphragm (4).

8. The bioreactor (1) according to any one of the preceding claims, further comprising cameras (16) for continuously and remotely controlling operation of the bioreactor (1) itself and/or sensors for continuously and remotely controlling operation of the bioreactor (1) itself.

9. The bioreactor (1) according to any one of the preceding claims, comprising two incubators (2), wherein each incubator (2) is connected to the same actuator (14b), said actuator being configured to produce a displacement of the fluid alternately towards one of the two incubators (2) and the other one of the two incubators (2).

10. A method for the recellularization of a diaphragm (4) **characterized by** a radial distribution of the muscle fibers, comprising:
- introducing a culture medium and the diaphragm into an incubator (2);
- placing the diaphragm (4) on a membrane (5);
- stressing the membrane (5) in order to stimulate the growth of the muscle fibers of the diaphragm in a radial direction on the first membrane (5),
wherein in order to stress the first membrane (5) the following steps are carried out:
providing a sealed body (14a) comprising a first chamber (11), a second chamber (13) and a connection duct (12) for connecting the first chamber (11) to the second chamber (13), wherein said first membrane (5) is a wall of said first chamber (11) and wherein said second chamber (13) includes at least a respective second membrane (15) or a respective flexible and/or deformable wall;
introducing a fluid inside the sealed body;
stressing the second membrane (15) of the second chamber (13) on an outer side of the sealed body and
producing a displacement of the fluid inside the sealed body (14a) from the second membrane (15) to the second surface (8) of the first membrane of the first chamber (11).

11. The method according to claim 10, wherein the pressure wave is applied centrally or in a centered manner with respect to the first membrane.

12. The method according to claim 10 or claim 11, wherein the first chamber (11) is arranged inside the incubator (2) and the second chamber (13) is arranged outside the incubator (2) and the connection duct (12) is a flexible pipe which is inserted inside a hole in a wall of the incubator (2).

13. The method according to any one of claims 10, 11 or 12, wherein at least two culture mediums provided with a respective diaphragm are prepared and wherein, using a same actuator alternately for each culture medium and for each diaphragm, said sealed body (14a) is prepared and the second membrane (15) is stressed and the displacement of the fluid contained inside said body is produced.

14. A bioreactor for implementing the method according to any one of claims 10 to 13.

15. The method for performing the recellularization of a diaphragm (4) by means of a bioreactor according to any one of claims 1 to 9.
